# EUROPÄISCHE PATENTSCHRIFT

(11) **EP 2 076 243 B1**
(45) Veröffentlichungstag und Bekanntmachung des Hinweises auf die Patenterteilung: **01.10.2014**
(21) Anmeldenummer: 08785714.0
(22) Anmeldetag: 27.08.2008
(51) Int. Cl.: A61K 9/08, A61K 38/19, A61K 47/12, A61K 47/26, A61P 35/00, A61P 7/00

(54) **FLÜSSIGFORMULIERUNG VON G-CSF**
LIQUID FORMULATION OF G-CSF
FORMULATION LIQUIDE DU G-CSF

(30) Priorität: 27.08.2007 EP 07016763; 27.08.2007 DE 102007040932
(43) Veröffentlichungstag der Anmeldung: 08.07.2009
(60) Teilanmeldung: 12162063.7
(73) Patentinhaber: ratiopharm GmbH, 89079 Ulm (DE)
(72) Erfinder: HINDERER, Walter, 63110 Rodgau (DE); LUBENAU, Heinz, 67435 Neustadt (DE)
(74) Vertreter: Steinecke, Peter
(86) Internationale Anmeldenummer: PCT/EP2008/007012
(87) Internationale Veröffentlichungsnummer: WO 2009/027076

(56) Entgegenhaltungen:
- WO-A-94/14466
- WO-A-2005/039620
- WO-A-2005/042024
- WO-A-2007/099145
- HERMAN A C ET AL: "CHAPTER 7: Characterization, formulation, and stability of Neupogen (Filgrastim), a recombinant human granulocyte-colony stimulating factor", 1 January 1996 (1996-01-01), FORMULATION, CHARACTERIZATION, AND STABILITY OF PROTEIN DRUGS: CASE HISTORIES,, PAGE(S) 303 - 328, XP009102549, ISBN: 978-0-306-45332-8
- RATIOPHARM PRESS RELEASE: 'Biosimilar Ratiograstim receives EMEA approval' September 2008,
- EMEA: 'Assessment Report for Ratiograstim' 2008,
- "Filgrastim" In: McEvoy, G.K. (ed.): "AHFS Drug Information 2001", 2001, American Society of Health System Pharmacists pages 1454-1463,

## Beschreibung

Gegenstand der vorliegenden Erfindung sind Arzneimittel zur Verwendung in der Behandlung von Krebs, schwerer chronischer Neutropenie (SCN), HIV-Infektion, der Schädigung des Zentralnervensystems oder von Nebenwirkungen aufgrund zytotoxischer Chemotherapie umfassend eine wässrige Flüssigformulierung von G-CSF im wesentlichen bestehend aus G-CSF und einem Zuckeralkohol, Polysorbat 80 in einer Konzentration von 0,05-0,06 mg/ml, Acetat in einer Konzentration von 5-20 mM als Puffersubstanz bei einem pH von 4,15-4,3, und gegebenenfalls Aminosäuren und/oder Glycerin und/oder Kohlenhydrate und/oder Konservierungsmittel, wobei das Acetat in Form seiner Essigsäure eingesetzt und der pH mit NaOH eingestellt wird.

Viele der bisher bekannten Arzneiformen für Proteinwirkstoffe besitzen Nachteile, beispielweise enthalten einige Zubereitungen pharmazeutische Zusatz- oder Hilfsstoffe, die aus medizinischer Sicht nicht ohne weiteres als unbedenklich einzustufen sind. Polymere und Proteine besitzen im Hinblick auf ihre Eignung als pharmazeutische Zusatzstoffe aufgrund ihrer Herkunft und ihrer physikalisch-chemischen Eigenschaften ein gewisses Risikopotential. Proteine menschlichen oder tierischen Ursprungs sowie aus Zellkulturen gewonnene Proteine tragen ein potentielles Restrisiko viraler Verunreinigungen. Auch andere, analytisch schwer nachweisbare proteinartige Verunreinigungen können wegen ihrer antigenen Eigenschaften immunologische Reaktionen beim Menschen hervorrufen. Proteine tierischen Ursprungs können darüber hinaus generell aufgrund ihrer spezies-spezifischen Eigenschaften immunologische Reaktionen beim Menschen auslösen. Auch Langzeitreaktionen nach späterer Re-Applikation derartiger Proteine sind möglich.

Der Zusatz von hochmolekularen Verbindungen kann ebenfalls problematisch sein. Polymere sind aufgrund ihrer großen Molekülmasse im Körper akkumulierbar und können somit, falls kein Bioabbau erfolgt, über lange Zeit im Körper verbleiben. Dies ist besonders bei subkutaner Applikation zu befürchten, da der Abtransport und die Verteilung durch den Blutstrom gegenüber intravenöser Gabe stark verlangsamt erfolgt. In Abhängigkeit von der Molmasse können Polymere auch antigene Eigenschaften aufweisen. Auch ist die Reinheit von Polymeren aufgrund der zur Herstellung verwendeten Katalysatoren oder des Vorhandenseins von Monomeren und anderen Polymer-Bruchstücken schwierig zu gewährleisten. Der Einsatz von Polymeren bei flüssigen pharmazeutischen Darreichungsformen, insbesondere bei subkutan applizierbaren Arzneiformen ist somit, wenn möglich, zu vermeiden.

Aus der Literatur ist ferner bekannt, dass insbesondere nicht glykosilierte Formen von G-CSF gegenüber glykosiliertem G-CSF, das aus CHO-Zellen gewonnen wird, aufgrund von Oxidation und/oder Aggregation besonders instabil sind. Die Stabilisierung von nicht glykosilierten Formen von G-CSF ist deshalb besonders schwierig und bedarf speziell ausgewählter Maßnahmen, um dieses Molekül in einer stabilen Arzneiform zu formulieren.

Der Einsatz von Tensiden zur Stabilisierung von G-CSF ist grundsätzlich aus medizinischer Sicht zu vermeiden, da sie lokale Irritationen auslösen können. Ebenso können Formulierungen mit sehr niedrigem pH-Wert insbesondere bei subkutaner Anwendung zu lokalen Unverträglichkeiten beim Patienten führen, beispielsweise zu Schmerzen und lokaler Gewebereizung, da der im Gewebe physiologisch vorliegende Bereich von pH 7,0-7,5 unterschritten wird.

In der jüngst veröffentlichten internationalen Anmeldung WO2005/042024 wird ähnlich wie in der EP 373 679 gelehrt, stabile pharmazeutische Zusammensetzungen von G-CSF zu erhalten, indem die Zusammensetzungen unter anderem frei von Tensiden gehalten werden und gemäß den in den Beispielen beschriebenen Zubereitungen der verwendete Puffer in einer sehr geringen Konzentration vorliegt. Angaben zur Verträglichkeit der beschriebenen Arzneimittel werden nicht gemacht.

In der europäischen Patentanmeldung EP 1 129 720 werden Zubereitungen von G-CSF mit einem pH-Bereich von 5 bis 8 beschrieben, wobei Sulfat das G-CSF in der Zusammensetzung stabilisieren soll. Auch hier werden keine Angaben zur Verträglichkeit der beschriebenen G-CSF-Zubereitungen gemacht.

In "Formulation, Characterisation, and Stability of Protein Drugs: Case Histories", Pearlman, Rodney, Wang, Y. John (Eds.), Seiten 303-328 wird die Zusammensetzung einer kommerziell erhältlichen G-CSF-Formulierung (Neupogen^{®}) beschrieben.

Aufgabe der vorliegenden Erfindung ist es, eine flüssige Arzneiform für G-CSF zur Verfügung zu stellen, die die oben beschriebenen Nachteile der bisher bekannten Arzneiformen nicht aufweist. Die pharmazeutische Zubereitung sollte insbesondere über einen längeren Zeitraum stabil und physiologisch gut verträglich sein. Sie sollte insbesondere für den Patienten zur Selbstanwendung geeignet sein, und sich durch die Vermeidung von oftmals mit der Selbstgabe von Arzneimitteln per Spritze oder Infusion auftretende Schmerzen an der Einstichstelle und unerwünschte Hautirritationen auszeichnen.

Gelöst wird diese Aufgabe durch die in den Ansprüchen gekennzeichneten und nachfolgend erläuterten Ausführungsformen. Insbesondere betrifft die vorliegende Erfindung Arzneimittel zur Verwendung in der Behandlung von Krebs, schwerer chronischer Neutropenie (SCN), HIV-Infektion, der Schädigung des Zentralnervensystems oder von Nebenwirkungen aufgrund zytotoxischer Chemotherapie umfassend eine wässrige Flüssigformulierung von G-CSF im wesentlichen bestehend aus G-CSF und einem Zuckeralkohol, Polysorbat 80 in einer Konzentration von 0,05-0,06 mg/ml, Acetat in einer Konzentration von 5-20 mM als Puffersubstanz bei einem pH von 4,15-4,3, und gegebenenfalls Aminosäuren und/oder Glycerin und/oder Kohlenhydrate und/oder Konservierungsmittel, wobei das Acetat in Form seiner Essigsäure eingesetzt und der pH mit NaOH eingestellt wird. Außerdem kann die erfindungsgemäße Flüssigformulierung weitere pharmazeutisch übliche Hilfsstoffe enthalten, bevorzugt ist jedoch die Abwesenheit dieser und/oder anderer Hilfsstoffe.

In der internationalen Anmeldung WO94/14466 werden allgemein lagerstabile wässrige pharmazeutische Zubereitungen von G-CSF beansprucht, die unter Verwendung von verschiedenen Puffersystemen in einem pH-Bereich von 3,5 bis 5 und 7 bis 8 stabil sein sollen. Allerdings werden nur flüssige G-CSF-Zubereitungen mit Phosphatpuffer auf ihre Langzeitstabilität getestet, während einzelne Versuchszubereitungen mit anderen Puffern lediglich nach kurzer mechanischer Belastung, das heißt, Turbidität auch das Auftreten von Trübungen in den Proben untersuchen wurden.

In Experimenten, die im Rahmen der vorliegenden Erfindung durchgeführt wurden musste dabei festgestellt werden, dass die in den Beispielen der WO94/14466 mit Acetatpuffer angegebenen G-CSF-Lösungen, insbesondere die Rezeptur 4 mit 10 mM Acetat und einem pH-Wert von 4,5 nicht die gewünschte Stabilität aufweist, da kritische Werte für die Anwesenheit von Aggregaten und oxidierten Formen von G-CSF ermittelt wurden. Dementsprechend wurde zunächst davon ausgegangen, dass wie bereits in der EP 0 373 679 erläutert, ein pH-Wert von über 4,0 zur Bildung von Aggregaten führt, und eine Flüssigformulierung von G-CSF unter Verwendung des ansonsten bevorzugten Acetatpuffersystems mit einem physiologisch verträglicheren pH-Wert nicht zu erzielen ist.

In der internationalen Anmeldung WO 2005/039620 wurden G-CSF-Flüssigformulierungen der Zusammensetzung 0,6 mg/ml G-CSF, 10 mM Acetatpuffer, 0,004 % (w/v) Tween 80 und 5 % (w/v) D-Sorbit mit einem pH-Wert von 4,0 und 4,2 als Vergleichsformulierungen in Untersuchungen zur Stabilität von G-CSF-Formulierungen bei mechanischem Stress bzw. nach Einfrieren und Auftauen von G-CSF-Formulierungen mit Succinatpuffer mit einem pH-Wert von 5,0 und 0,02 % (w/v) Tween 20 gegenübergestellt. Nach diesen Untersuchungen waren die acetatgepufferten G-CSF-Formulierungen instabiler, so dass es nicht sinnvoll erschien, solche Acetat-gepufferten Zusammensetzungen weiter zu untersuchen, geschweige denn als Arzneimittel in Betracht zu ziehen.

Dagegen wurde in Experimenten, die im Rahmen der vorliegenden Erfindung durchgeführt wurden, überraschenderweise vorteilhafte Eigenschaften von Acetat-gepufferten G-CSF-Formulierungen mit pH > 4.0 bezüglich Stabilität bei Lagertemperatur im Kühlschrank und bei 40°C gefunden, das heißt im Bereich der Körpertemperatur sowie unerwartet besonders gute Verträglichkeit bei Patienten, da gegenüber handelsüblichen Arzneimitteln weniger Hautirritationen auftraten."

Insbesondere wurde gefunden, dass man im Sinne der vorliegenden Erfindung durch Kombination von Essigsäure bzw. Acetat als Puffersubstanz und einem Tensid wie Polysorbat 80 in jeweils einer bestimmten Konzentration nebst Anwesenheit eines Zuckeralkohols wie Sorbit und Einstellung des pH-Werts auf etwa 4,2 ± 0,15 eine stabile Flüssigformulierung von G-CSF erhalten wird, die den G-CSF-Molekülen die für deren Arzneimitteltauglichkeit erforderliche Stabilität verleiht, gut verträglich ist und zusätzlich Symptome, die häufig bei Verabreichung des Arzneimittels an der Einstichstelle zu beobachten sind, fast vollständig vermeidet. Dies macht die erfindungsgemäßen G-CSF-Flüssigformulierungen in Fertigspritzen und damit hergerichteten Kits insbesondere für den Heimgebrauch geeignet und vorteilhaft.

Die erfindungsgemäßen Flüssigformulierungen besitzen ferner den Vorteil, dass sie vorzugsweise frei von proteinartigen oder polymeren Hilfsstoffen sind, deren Verwendung aus medizinischer Sicht problematisch sein kann. Wie durch die in den Beispielen erläuterten klinischen Studien gezeigt, besitzen sie ferner den Vorteil, gut verträglich und weitgehend schmerzfrei applizierbar zu sein. Die erfindungsgemäße G-CSF Flüssigformulierung ist dabei vorzugsweise auch im Wesentlichen frei von Aminosäuren und/oder zusätzlichen Proteinen wie Serumalbumin. In einer Ausführungsform ist die erfindungsgemäße G-CSF Flüssigformulierung frei von Methionin.

Vorteilhaft ist ferner, dass sich in den biochemischen Experimenten und klinischen Studien herausgestellt hat, dass aufgrund der getroffenen Auswahl der Puffersubstanz in einem bestimmten Konzentrationsbereich und die Anwesenheit des Zuckeralkohols niedrige Tensidmengen von etwa 0,05 bis 0,06 mg/ml zum einen ausreichend zur Stabilisierung von G-CSF sind, und zum anderen keine wesentlichen Hautirritationen oder sonstigen Unverträglichkeit beim Patienten hervorrufen. Dies ist vor allem bei solchen flüssigen Arzneiformen von Vorteil, die für die subkutane Anwendung von G-CSF bestimmt sind. Außerdem werden durch die erfindungsgemäßen Maßnahmen insbesondere die labilen, unglykosilierten G-CSF-Moleküle für pharmazeutische Zubereitungen ausreichend stabilisiert. Durch die gezielt getroffene Auswahl der Hilfsstoffe werden insgesamt sehr gut verträgliche G-CSF-haltige flüssige Arzneiformen zur Verfügung gestellt, die bezüglich der Proteinstabilität qualitativ hochwertige Zubereitungen darstellen, und insbesondere als fertige Injektions- oder Infusionslösungen geeignet sind.

In den erfindungsgemäßen Flüssigformulierungen werden Tensidmengen von etwa 0,05 bis 0,06 mg/ml verwendet, besonders bevorzugt etwa 0,06 mg/ml. Das verwendete Tensid ist Polysorbat 80, auch bekannt als Tween 80©.

Die erfindungsgemäßen G-CSF-haltigen Arzneiformen enthalten den Wirkstoff in einer zur Erzielung eines therapeutischen Effektes ausreichenden Menge. In der Regel werden Wirkstoffkonzentrationen von 0,01-5 mg/ml verwendet, vorzugsweise 0,1-1 mg/ml, 0,3 bis 0,8 mg/ml und besonders bevorzugt eine Konzentration von etwa 0,6 mg/ml. Dabei haben sich insbesondere bei der subkutanen Applikation Dosisstärken von 0,3 mg/0,5 ml und 0,48 mg /0,8 ml als besonders bevorzugt herausgestellt.

Erfindungsgemäß wird als Puffersubstanz Essigsäure eingesetzt. Bei der Herstellung der erfindungsgemäßen Flüssigformulierung wird die Puffersubstanz in Form ihrer freien Säure vorgegeben. Der gewünschte pH-Wert der Lösung wird durch Zugabe von Basen, wie beispielsweise von Alkalihydroxiden, Erdalkalihydroxiden oder Ammoniumhydroxid eingestellt. Vorzugsweise wird hierzu Natriumhydroxid verwendet.

Die Konzentrationen der Puffersubstanz Essigsäure in der fertig applizierbaren flüssigen Arzneiform beträgt etwa 5-20 mMol/l; der Einfachheit halber wird nachfolgend Bezug genommen auf die Anionen- Konzentrationen dieser Säure, also Acetat, wobei auch nicht dissoziierte Essigsäure von dem Begriff Acetat umfasst werden soll. Bevorzugt werden folgende Pufferkonzentrationen und pH-Werte verwendet: 7,5-15 mMol, besonders bevorzugt 10 mMol Acetat und pH 4,15 bis 4,3; und insbesondere pH 4,2.

Das in den erfindungsgemäßen Flüssigformulierungen verwendete G-CSF bezieht sich prinzipiell auf alle durch rekombinante Verfahren hergestellte G-CSF-Moleküle und deren Varianten. Der Begriff G-CSF oder G-CSF-Variante gemäß vorliegender Erfindung beinhaltet alle natürlich vorkommenden Varianten von G-CSF, sowie davon abgeleitete durch rekombinante DNA-Technologie modifizierten G-CSF-Proteine, insbesondere Fusiorisproteine, die neben dem G-CSF-Anteil noch andere Proteinsequenzen enthalten. Besonders bevorzugt ist in diesem Sinne ein G-CSF-Mutein mit einem N-terminalen Met-Rest an Position -1, das durch die Expression in prokaryontischen Zellen entsteht. Ebenso geeignet ist eine rekombinante methioninfreie G-CSF-Variante, die gemäß der WO91/11520 hergestellt werden kann. Unter dem Begriff "G-CSF-Variante" werden solche G-CSF-Moleküle verstanden, bei denen eine oder mehrere Aminosäuren deletiert oder durch andere Aminosäuren ersetzt sein können, wobei die wesentlichen Eigenschaften von G-CSF weitgehend erhalten bleiben. Geeignete G-CSF-Muteine sind beispielsweise in EP 0 456 200 beschrieben. Besonders bevorzugt ist das in den erfindungsgemäßen Flüssigformulierungen vorliegende G-CSF nicht glykosiliert.

Zur Herstellung gut verträglicher parenteraler Arzneiformen ist der Zusatz von isotonisierenden Hilfsstoffen zweckmäßig, wenn nicht durch die osmotischen Eigenschaften des Wirkstoffes und der zur Stabilisierung eingesetzten Hilfsstoffe bereits Isotonie erreicht werden kann. Dazu werden vor allem nicht-ionisierte, gut verträgliche Hilfsstoffe, wie z. B. Mannit, Glycerin oder andere Zuckeralkohole eingesetzt. Im Falle von G-CSF wird vorzugsweise Sorbit eingesetzt, das besonders vorteilhaft für die Verträglichkeit der erfindungsgemäßen Flüssigformulierungen ist. Vorzugsweise liegt der Zuckeralkohol in der Flüssigformulierung in einer Konzentration von etwa 2,5 bis 7,5% (w/v) vor, besonders bevorzugt in einer Konzentration von etwa 5% (w/v).

Der Zusatz von Salzen ist zur Einstellung der Isotonie nicht vorteilhaft, da hohe Salz- oder Ionenkonzentrationen die Aggregatbildung von G-CSF fördern. Vorteilhaft werden deshalb Salze in geringer Menge zugesetzt. Die Pufferkonzentrationen sind so bemessen, dass der pHstabilisierende Effekt zwar erreicht, die Ionenstärke jedoch so gering als möglich gehalten wird. Die Pufferkonzentrationen liegen vorzugsweise im Bereich von bis zu 20 mMol, insbesondere weniger als 15 mMol.

Außerdem können die fertig applizierbaren Injektionslösungen weitere übliche Hilfs- oder Zusatzstoffe enthalten. Es können Antioxidantien, wie beispielsweise Glutathion, Ascorbinsäure oder ähnliche Substanzen, chaotrope Hilfsstoffe, wie beispielsweise Harnstoff oder Aminosäuren, wie beispielsweise Methionin, Arginin, Lysin, Ornithin, u. a. zugesetzt werden.

In einer besonders bevorzugten Ausführungsform ist die erfindungsgemäße wässrige Flüssigformulierung von G-CSF als Injektions- oder Infusionslösung hergerichtet und besteht im Wesentlichen aus humanem, nicht-glykolisiertem methionyl-G-CSF in einer Konzentration von etwa 0,6 mg/ml und Sorbitol in einer Konzentration von etwa 5% (w/v), Polysorbat 80 in einer Konzentration von etwa 0,06 mg/ml, und Acetat in einer Konzentration von etwa 10 mM als Puffersubstanz bei einem pH von etwa 4,2.

Ansonsten können die erfindungsgemäßen Flüssigformulierungen von G-CSF bei gleicher Dosisstärke entsprechend den Gebrauchsinformationen für Neupogen© verwendet werden, insbesondere bezüglich Dosierung, Verabreichung, und medizinischer Indikation da in erfindungsgemäßen Flüssigformulierungen vorzugsweise ebenfalls hochgereinigtes, nichtglykosiliertes G-CSF-Protein wie Filgrastim verwendet wird, das in einem Laborstamm von *E. coli* K12 hergestellt wird.

Die erfindungsgemäßen Flüssigformulierungen werden als Arzneimittel zur Behandlung von Krankheiten eingesetzt, für die bekannt ist, dass sie durch Verabreichung von G-CSF behandelbar sind, wie Krebs, Nebenwirkungen aufgrund zytotoxischer Chemotherapie, begleitende Therapie von Krankheiten, in der eine periphere Blutvorläuferzell-Mobilisierung vonnöten ist, schwere chronische Neutropenie (SCN), HIV-Infektion und andere. Dementsprechend betrifft die vorliegende Erfindung Arzneimittel umfassend eine der vorstehend beschriebenen G-CSF-Flüssigformulierungen.

In einer Ausführungsform ist die erfindungsgemäße G-CSF-Zusammensetzung zur Herstellung eines Arzneimittels vorgesehen, zur Behandlung neurologischer Indikationen wie Schädigung des Zentralnervensystems nach beispielsweise akutem Schlaganfall, bei denen bei frühzeitiger Gabe, beispielsweise als Bolus, schwere sekundäre Schädigungen gemildert oder vermieden werden können. Ansonsten sind die Flüssigformulierungen der vorliegenden Erfindung als Arzneimittel vorzugsweise zur subkutanen oder intravenösen Verabreichung vorgesehen, beispielsweise durch Injektion mit vorgefüllten Spritzen oder durch Infusion, wenn eine längere kontinuierliche Gabe von G-CSF wünschenswert ist.

Wie bereits vorstehend erläutert, ist die erfindungsgemäße Flüssigformulierung von G-CSF über einen langen Zeitraum stabil und kann grundsätzlich in jedem geeigneten Gefäß aufbewahrt werden. Dementsprechend betrifft die vorliegende Erfindung auch ein Gefäß, enthaltend eine der vorstehend oder in den Beispielen beschriebene erfindungsgemäße Flüssigformulierung von G-CSF. Vorzugsweise ist mindestens eine Oberfläche des Gefäßes, die in Kontakt mit der Flüssigformulierung ist, mit einem Material bestehend aus Silikon oder Polytetrafluorethylen bzw. Ethylen-Tetrafluorethylen (ETFE) beschichtet. Typischerweise handelt es sich bei dem Gefäß um Behälter die üblicherweise für die Aufbewahrung und/oder Verabreichung von Flüssigarzneimittel bestimmt sind wie Vial, Spritze, Ampulle, Karpule oder Infusionsbehälter, wobei die erfindungsgemäße Flüssigformulierung von G-CSF insbesondere vorteilhaft für die Verwendung in Fertigspritzen und Ampullen ist. In einer bevorzugten Ausführungsform liegt die Flüssigformulierung in der Spritze oder Ampulle bezüglich der Konzentration von G-CSF in einer Konzentration von 0,3 mg in 0,5 ml oder 0,48 mg in 0,8 ml vor.

Da der erfindungsgemäßen Flüssigformulierung von G-CSF vorteilhafterweise keine weiteren Hilfsstoffe vor deren Verabreichung zugesetzt werden, oder andere vorbereitende Maßnahmen ergriffen werden müssen wie Filtrieren, Mischen, etc. kann das erfindungsgemäße G-CSF-Flüssigarzneimittel unmittelbar zur dessen Verabreichung hergerichtet werden, beispielsweise in einem Kit.

In eine für den Arzt, Apotheker und insbesondere Patienten besonders vorteilhaften Ausführungsform betrifft die vorliegende Erfindung daher auch ein Kit zur parenteralen Verabreichung von G-CSF umfassend ein oder mehrere der vorstehend beschriebenen Gefäße vorzugsweise nebst Anweisungen zur Lagerung und/oder Verabreichung. Üblicherweise wird die Verabreichung von G-CSF mit einer Dosis von 5-30 µg/kg Körpergewicht vorgesehen sein, wobei allerdings je nach medizinischer Indikation und Stadium der Krankheit auch niedrigere oder höhere Dosen angezeigt sein können.

In dem erfindungsgemäßen Kit sind vorzugsweise 5 Spritzen oder Ampullen vorgesehen, ggf. aber auch mehr wie jeweils 7, beispielweise wenn die tägliche Verabreichung über eine Woche vorgesehen ist.

Zur sicheren Handhabung verfügt das erfindungsgemäße Kit vorteilhafterweise über Sicherheitskompartimente für Spritzen, beziehungsweise für Injektions- und/oder Infusionskanülen. Hierbei kommen auch Abwurflülfen für die Kanülen und vorbereitete wie vorgesteckte Verschlusskappen in Betracht.

Wie in den Beispielen beschrieben sind die erfindungsgemäßen G-CSF-Flüssigformulierungen insbesondere bei ca. 5°C über einen längeren Zeitraum stabil, vorzugsweise mindestens 4 Wochen. Daher lassen sich die erfindungsgemäßen Flüssigformulierungen, Gefäße und Kits vorteilhafterweise im normalen Kühlschrank lagern.

Techniken zur Ausführung der Erfindung sind dem Fachmann bekannt und in der einschlägigen Literatur beschrieben, siehe beispielsweise Molecular Cloning A Laboratory Manual, 2nd Ed., ed. by Sambrook, Fritsch and Maniatis (Cold Spring Harbor Laboratory Press: 1989); Immunochemical Methods In Cell And Molecular Biology (Mayer and Walker, eds., Academic Press, London, 1987); Handbook Of Experimental Immunology, Volumes I-IV (D. M. Weir and C. C. Blackwell, eds., 1986).

### Beispiel

Im folgenden wird die Erfindung anhand von einem bevorzugten Ausführungsbeispiel näher beschrieben, ohne jedoch den Gegenstand der vorliegenden Erfindung einzuschränken.

### Beispiel 1: Herstellung der flüssigen G-CSF Formulierungen

Die in den Beispielen verwendeten Lösungen von G-CSF wurden hergestellt, indem die beschriebenen Hilfsstoffe in Wasser für Injektionszwecke gelöst wurden, G-CSF in der angegebenen Menge zugesetzt wurde und, falls notwendig, der pH-Wert mit einer kleinen Menge Pufferkomponente genau auf den Sollwert eingestellt wurde.

Bei der Herstellung der erfindungsgemäßen G-CSF-Formulierung ist darauf zu achten, dass zur Einstellung des Acetatpuffers zunächst Essigsäure vorgelegt wird und der pH-Wert mit einer NaOH-Lösung eingestellt wird, da sich in Vorexperimenten herausgestellt hat, dass bei Verwendung von Natriumacetat und nachfolgender Einstellung der pH-Werte mit HCl es zu Proteinaggregationen kommen kann, womöglich aufgrund einer erhöhten Ionenkonzentration.

**Tabelle 1: pH-Werte in Flüssigformulierungen mit einer Dosisstärke von 30 MIU, 0,6 mg/ml, 0,5 ml/Ampulle während der Lagerung für 4 Wochen bei +40°C und +5°C.**

| **Notierung** | **Bezeichnung der Zusammensetzung** | Anfangswert +5°C | 1. Woche bei +40°C | 2. Woche bei +40°C | 4. Woche bei +40°C | 4. Woche bei +5°C |
|---|---|---|---|---|---|---|
| | | pH | pH | pH | pH | pH |
| I | 10 mM Essigsäure, pH 4.1; 5% Sorbit, 0.004% Polysorbat 80 | 4.05 | 4.08 | 4.10 | 4.12 | 4.07 |
| IIa | 10 mM Essigsäure, pH 4.2; 5% Sorbit; 0.004% Polysorbat 80 | 4.23 | 4.24 | 4.24 | 4.25 | 4.23 |
| IIb | 10mM Essigsäure, pH 4.3; 5% Sorbit; 0.004% Polysorbat 80 | 4.33 | 4.35 | 4.34 | 4.36 | 4.33 |
| IIc | 10 mM Essigsäure, pH 4.4; 5% Sorbit; 0.004% Polysorbat 80 | 4.43 | 4.45 | 4.43 | 4.47 | 4.43 |
| IIIa | 10 mM Essigsäure, pH 4.2; 5% Sorbit; 0.006% Polysorbat 80 | 4.24 | 4.24 | 4.24 | 4.27 | 4.23 |
| IIIb | 10 mM Essigsäure, pH4.3; 5% Sorbit; 0.006% Polysorbat 80 | 4.34 | 4.34 | 4.35 | 4.37 | 4.33 |
| IIIc | 10mM Essigsäure, pH 4.4; 5% Sorbit; 0.006% Polysorbat 80 | 4.43 | 4.45 | 4.43 | 4.47 | 4.43 |
| | | | | | | |
| IVa | 10mM Essigsäure, pH 4.2; 5% Sorbit; 0.008% Polysorbat 80 | 4.21 | 4.24 | 4.22 | 4.25 | 4.21 |
| IVb | 10mM Essigsäure, pH 4.3; 5% Sorbit; 0.008% Polysorbat 80 | 4.33 | 4.33 | 4.34 | 4.36 | 4.31 |
| IVc | 10mMEssigsäure,pH4.4;5%Sorbit; 0.008% Polysorbat 80 | 4.43 | 4.45 | 4.43 | 4.45 | 4.41 |

Die Lösungen werden dann durch geeignete sterilisierte Membranfilter von 0,2 m Porenweite filtriert und in sterilisierte Injektionsfläschlein aus Glas der hydrolytischen Klasse 1 abgefüllt und mit sterilen, teflonisierten Gummistopfen verschlossen. Die Abfüllung erfolgt vorzugsweise unter Stickstoffbegasung.

### Beispiel 2: Biochemische Stabilität der erfindungsgemäßen G-CSF Formulierungen

Die Versuchszubereitungen von G-CSF werden in verschlossenen und verbördelten Flaschen unter Lichtausschluss bei definierten Lagertemperaturen gelagert und danach auf Proteinreinheit sowie das Auftreten von oxidierten Formen, Aggregaten und Dimeren hin unter Verwendung von Standardtechniken untersucht wie UV-Spektroskopie, reversed-phase-HPLC auf C4 und C18 stationärer Phase, oder size exclusion Chromatographie (SEC HPLC), Isoelektrofokussierung, SDS-PAGE unter Thiol-reduzierenden und Thiol-nichtreduzierenden Bedingungen mit anschließender Silberfärbung und Western lmmunoblot-Analysen; siehe zu den Techniken beispielsweise die Ausführungsbeispiele der WO94/14466.

Die in der Tabelle 1 von Beispiel 1 angegebenen Flüssigformulierungen von G-CSF wurden auf ihre Langzeitstabilität wie angegeben getestet. Für die Formulierungen mit einem Tensidgehalt von > 0,006% wird beispielhaft lediglich die mit 0,008% angegeben, da Formulierungen mit höherem Tensidgehalt eine noch wesentlich schlechtere Langzeitstabilität als diese aufwiesen. Bei Experimenten zur biochemischen Stabilität von G-CSF in den Versuchszusammensetzungen stellte sich heraus, dass bei Einsatz von höheren Tensidkonzentrationen bis herunter zu einem Gehalt von einschließlich 0,008% in großer Menge die Akkumulierung der oxidierten Form von G-CSF zu beobachten war. Daher wurde in den Experimenten, die im Rahmen der vorliegenden Erfindung durchgeführt wurden, entgegen der Lehre in der internationalen Anmeldung WO94/14466 festgestellt, dass selbst geringere Tensidkonzentration, in jedem Falle aber hohe Tensidkonzentration negative Auswirkungen auf das Oxidationsprofil von G-CSF in Lösung hat.

Ferner wurde festgestellt, dass sich die in Tabelle 1 angegebenen erfindungsgemäßen G-CSF-Formulierungen dadurch auszeichnen, dass der relative Gehalt an oxidierten Spezies unter 1 % liegt, während dieser Schwellenwert bei den Kontrollproben mit einem Tensidgehalt von 0,008% und mehr überschritten wurde und solche Lösungen daher nicht als Arzneimittel in Betracht kommen.

Weitergehende Untersuchungen zur biochemischen Stabilität von G-CSF in den Versuchszusammensetzungen ergaben, dass die erfindungsgemäßen Formulierungen sowohl die Aggregation als auch die Oxidation von G-CSF wesentlich verhindern können, ohne dass es eines weiteren Zusatzes von Hilfsstoffen wie Aminosäuren und/oder Antioxidanzien bedarf.

### Beispiel 3: Verträglichkeit der G-CSF Formulierungen

Eine der erfindungsgemäßen Flüssigformulierungen wurde in drei klinischen Studien der Phase III an Krebspatienten getestet, wobei unter anderem die Reaktionen der Patienten an der Einstichstelle untersucht wurden. Hierbei wurden Schwellungen, Rötungen, Hautblutungen, Druckempfindlichkeit und andere Symptome an der Einstichstelle untersucht. Für die klinischen Studien wurde die erfindungsgemäße G-CSF-Formulierung entsprechend den Angaben zur Versuchszubereitung IIa (XM02) in Tabelle 1 ausgewählt und mit einer Dosierung von 5 µg/kg Körpergewicht pro Tag verarbeitet

Bei diesen Untersuchungen stellte sich erfreulicherweise heraus, dass bei einer Gesamtzahl von 356 Patienten lediglich ein Patient unerwünschte Reaktionen berichtete, das heißt, in lediglich 0,3% der Fälle. In Kontrollexperimenten mit der auf dem Markt erhältlichen G-CSF-Formulierung von Neupogen© konnte gezeigt werden, dass die erfindungsgemäße G-CSF-Formulierung eine um den Faktor 4 bessere Verträglichkeit bezüglich der Nebenreaktion an der Einstichstelle bei Patienten aufweist. Diese Ergebnisse sind in der nachfolgenden Tabelle zusammengefasst.

**Tabelle 2: Häufigkeit von Reaktionen an der Einstichstelle in drei klinischen Phase III-Studien. Auswertung des ersten Zyklus der Chemotherapie von Brustkrebs, Non-Hodgkin-Lymphom und Lungenkrebs**

| **Medikation** | **pH** | **Gesamtzahl** | **Positive** | **% Positive** |
|---|---|---|---|---|
| XM02 | 4,2 | 356 | 1 | 0,3 |
| Neupogen | 4,0 | 249 | 3 | 1,2 |
| Placebo | 7,0 | 72 | 0 | 0,0 |

Aus den vorstehenden Untersuchungen ergibt sich, dass durch Kombination von Essigsäure bzw. Acetat als Puffersubstanz und einem Tensid wie Polysorbat 80 in jeweils einer bestimmten Konzentration nebst Anwesenheit eines Zuckeralkohols wie Sorbit und Einstellung des pH-Werts auf etwa 4,2 ± 0,15 eine stabile Flüssigformulierung von G-CSF erhalten wird, die den G-CSF-Molekülen die für deren Arzneimitteltauglichkeit erforderliche Stabilität verleiht, gut verträglich ist und zusätzliche Symptome, die bei Verabreichung des Arzneimittels an der Einstichstelle zu beobachten sind, fast vollständig vermeidet. Dies macht die erfindungsgemäßen G-CSF-Flüssigformulierungen bzw. daraus hergerichtete Kits insbesondere für den Heimgebrauch geeignet und vorteilhaft.

## Patentansprüche

1. Arzneimittel zur Verwendung in der Behandlung von Krebs, schwerer chronischer Neutropenie (SCN), HIV-Infektion, der Schädigung des Zentralnervensystems oder von Nebenwirkungen aufgrund zytotoxischer Chemotherapie umfassend eine wässrige Flüssigformulierung von G-CSF im wesentlichen bestehend aus G-CSF und einem Zuckeralkohol, Polysorbat 80 in einer Konzentration von 0,05-0,06 mg/ml, Acetat in einer Konzentration von 5-20 mM als Puffersubstanz bei einem pH von 4,15-4,3, und gegebenenfalls Aminosäuren und/oder Glycerin und/oder Kohlenhydrate und/oder Konservierungsmittel, wobei das Acetat in Form seiner Essigsäure eingesetzt und der pH mit NaOH eingestellt wird.

2. Arzneimittel nach Anspruch 1, wobei das G-CSF nicht glykosiliert ist.

3. Arzneimittel nach Anspruch 1 oder 2, wobei das G-CSF in einer Konzentration von 0,6 mg/ml vorliegt.

4. Arzneimittel nach einem der Ansprüche 1 bis 3, wobei der Zuckeralkohol Sorbit ist.

5. Arzneimittel nach einem der Ansprüche 1 bis 4, wobei der Zuckeralkohol in einer Konzentration von 5% (w/v) vorliegt.

6. Arzneimittel nach einem der Ansprüche 1 bis 5, wobei das Acetat in einer Konzentration von 10 mM vorliegt.

7. Arzneimittel nach einem der Ansprüche 1 bis 6, die im Wesentlichen frei von Aminosäuren und/oder zusätzlichem Protein ist.

8. Arzneimittel nach einem der Ansprüche 1 bis 7, die eine Injektions- oder Infusionslösung ist.

9. Arzneimittel nach Anspruch 1 als Injektions- oder Infusionslösung, wobei das G-CSF humanes, nicht glykolisiertes Methionyl-G-CSF ist und in einer Konzentration von 0,6 mg/ml vorliegt, der Zuckeralkohol Sorbitol ist und in einer Konzentration von 5 % (w/v) vorliegt, das Tensid Polysorbat 80 ist und Acetat als Puffersubstanz in einer Konzentration von 10 mM vorliegt.

10. Gefäß zur Verabreichung von Flüssigarzneimitteln, enthaltend eine Arzneiflüssigformulierung nach einem der Ansprüche 1 bis 9.

11. Gefäß nach Anspruch 10, wobei mindestens eine Oberfläche des Gefäßes, die in Kontakt mit der Flüssigformulierung ist, mit einem Material bestehend aus Silikon oder Polytetrafluorethylen oder einem Ethylen-Tetrafluorethylen (ETFE) Copolymer beschichtet ist.

12. Gefäß nach Anspruch 10 oder 11, das eine Spritze, Ampulle, Karpule oder Infusionsbehälter ist.

13. Spritze oder Ampulle nach Anspruch 12, wobei die Flüssigformulierung bezüglich der Konzentration von G-CSF in einer Konzentration von 0,3 mg in 0,5 ml oder 0,48 mg in 0,8 ml vorliegt.

14. Kit zur parenteralen Verabreichung von G-CSF umfassend ein Gefäß nach einem der Ansprüche 10 bis 13 und Anweisungen zur Lagerung und/oder Verabreichung.

15. Kit nach Anspruch 14, wobei die Verabreichung von G-CSF mit einer Dosis von 5-30 µg/kg Körpergewicht vorgesehen ist.

16. Kit nach Anspruch 14 oder 15, wobei in dem Kit 5 Spritzen oder Ampullen vorgesehen sind.

17. Kit nach einem der Ansprüche 14 bis 16, das über Sicherheitskompartimente für Spritzen, beziehungsweise Injektions- und/oder Infusionskanülen verfügt.

18. Kit nach einem der Ansprüche 14 bis 17, wobei die Lagerung bei 5°C vorgesehen ist.

## Claims

1. Medicament for the use in the treatment of cancer, severe chronic neutropenia (SCN), HIV infection, damage of the central nervous system or of adverse side effects owing to cytotoxic chemotherapy comprising an aqueous liquid formulation of G-CSF substantially consisting of G-CSF and a sugar alcohol, polysorbate 80 at a concentration of 0.05-0.06 mg/ml, acetate at a concentration of 5-20 mM as a buffer substance at pH of 4.15-4.3, and optionally amino acids and/or glycerol and/or carbohydrates and/or preservatives, wherein acetate is added in the form of its acetic acid and the pH is adjusted with NaOH.

2. Medicament according to claim 1, wherein the G-CSF is non-glycosylated.

3. Medicament according to claim 1 or 2, wherein the G-CSF is present at a concentration of 0.6 mg/ml.

4. Medicament according to any one of claims 1 to 3, wherein the sugar alcohol is sorbitol.

5. Medicament according to any one of claims 1 to 4, wherein the sugar alcohol is present at a concentration of 5% (w/v).

6. Medicament according to any one of claims 1 to 5, wherein the acetate is present at a concentration of 10 mM.

7. Medicament according to any one of claims 1 to 6 which is substantially free of amino acids and/or additional protein.

8. Medicament according to any one of claims 1 to 7 which is an injection or infusion solution.

9. Medicament according to claim 1 as injection or infusion solution, wherein the G-CSF is human, non-glycosylated methionyl-G-CSF and is present at a concentration of 0.6 mg/ml, sugar alcohol is sorbitol and is present at a concentration of 5 % (w/v), surfactant is polysorbate 80 and acetate is present as buffer substance at a concentration of 10 mM.

10. Receptacle for the administration of liquid medicaments, comprising a liquid medicament formulation according to any one of claims 1 to 9.

11. Receptacle according to claim 10, wherein at least one surface of said receptacle, which is in contact with the liquid formulation, is coated with a material consisting of silicone or polytetrafluoroethylene or an ethylene-tetrafluoroethylene (ETFE) copolymer.

12. Receptacle according to claim 10 or 11 which is a syringe, an ampoule, a carpule or an infusion container.

13. Syringe or ampoule according to claim 12, wherein the liquid formulation is present, relating to the concentration of G-CSF, at a concentration of 0.3 mg in 0.5 ml or 0.48 mg in 0.8 ml.

14. Kit for the parenteral administration of G-CSF, comprising a receptacle according to any one of claims 10 to 13 as well as instructions for storage and/or administration.

15. Kit according to claim 14, wherein the administration of G-CSF is provided at a dosage of 5-30 µg/kg body weight.

16. Kit according to claim 14 or 15, wherein 5 syringes or ampoules are provided in said kit.

17. Kit according to any one of claims 14 to 16, wherein safety compartments for syringes or injection and/or infusion needles are provided.

18. Kit according to any one of claims 14 to 17, wherein storage is intended at 5°C.

## Revendications

1. Médicament pour utilisation dans le traitement du cancer, de la neutropénie sévère chronique (SCN), d'une infection par le VIH, de l'atteinte du système nerveux central ou d'effets secondaires issus d'une chimiothérapie cytotoxique, comprenant une formulation liquide aqueuse de G-CSF essentiellement constituée de G-CSF et d'un sucre-alcool, de polysorbate 80 à une concentration de 0,05 à 0,06 mg/ml, d'acétate à une concentration de 5 à 20 mM en tant que substance tampon à un pH de 4,15 à 4,3, et éventuellement d'acides aminés et/ou de glycérine et/ou d'hydrates de carbone et/ou de conservateurs, dans laquelle l'acétate est sous forme d'acide acétique et le pH est réglé avec NaOH.

2. Médicament selon la revendication 1, dans lequel le G-CSF n'est pas glycosylé.

3. Médicament selon la revendication 1 ou 2, dans lequel le G-CSF est présent à une concentration de 0,6 mg/ml.

4. Médicament selon l'une des revendications 1 à 3, dans lequel le sucre-alcool est le sorbitol.

5. Médicament selon l'une des revendications 1 à 4, dans lequel le sucre-alcool est présent à une concentration de 5 % (p/v).

6. Médicament selon l'une des revendications 1 à 5, dans lequel l'acétate est présent à une concentration de 10 mM.

7. Médicament selon l'une des revendications 1 à 6, qui est essentiellement exempt d'acides aminés et/ou de protéine additionnelle.

8. Médicament selon l'une des revendications 1 à 7, qui est une solution pour injection ou perfusion.

9. Médicament selon la revendication 1 en tant que solution pour injection ou perfusion, dans lequel le G-CSF est du G-CSF humain méthionylé, non glycosylé et est présent à une concentration de 0,6 mg/ml, le sucre-alcool est le sorbitol et est présent à une concentration de 5 % (p/v), le tensioactif est le polysorbate 80 et l'acétate est présent en tant que substance tampon à une concentration de 10 mM.

10. Récipient pour l'administration de médicaments liquides, contenant une formulation liquide de médicament selon l'une des revendications 1 à 9.

11. Récipient selon la revendication 10, dans lequel au moins une surface du récipient, qui est en contact avec la formulation liquide, est enduite d'un matériau constitué de silicone ou de polytétrafluoroéthylène ou d'un copolymère d'éthylène-tétrafluoroéthylène (ETFE).

12. Récipient selon la revendication 10 ou 11, qui est une seringue, une ampoule, une carpule ou un récipient pour perfusion.

13. Seringue ou ampoule selon la revendication 12, dans laquelle la formulation liquide présente une concentration en G-CSF de 0,3 mg dans 0,5 ml ou de 0,48 mg dans 0,8 ml.

14. Kit pour l'administration par voie parentérale de G-CSF comprenant un récipient selon l'une des revendications 10 à 13 et des instructions pour le stockage et/ou l'administration.

15. Kit selon la revendication 14, dans lequel l'administration de G-CSF est prévue à une dose de 5 à 30 µg/kg de poids corporel.

16. Kit selon la revendication 14 ou 15, dans lequel 5 seringues ou ampoules sont prévues dans le kit.

17. Kit selon l'une des revendications 14 à 16, qui dispose de compartiments de sécurité pour seringues, ou pour canules d'injection et/ou de perfusion.

18. Kit selon l'une des revendications 14 à 17, dans lequel le stockage est prévu à 5°C.
